# NEUE EUROPÄISCHE PATENTSCHRIFT

(11) **EP 3 139 877 B2**
(45) Veröffentlichungstag und Bekanntmachung des Hinweises auf die Entscheidung über den Einspruch: **05.07.2023**
(45) Hinweis auf die Patenterteilung: 04.07.2018
(21) Anmeldenummer: 15720670.7
(22) Anmeldetag: 28.04.2015
(51) Int. Cl.: A61F 13/00

(54) **SCHAUMWUNDAUFLAGE FÜR DIE UNTERDRUCKTHERAPIE**
FOAM-TYPE WOUND DRESSING FOR VACUUM THERAPY
PANSEMENT EN MOUSSE POUR TRAITEMENT SOUS VIDE

(30) Priorität: 09.05.2014 DE 102014106518
(43) Veröffentlichungstag der Anmeldung: 15.03.2017
(73) Patentinhaber: Paul Hartmann AG, 89522 Heidenheim (DE)
(72) Erfinder: DEIBLER, Martin, 89542 Herbrechtingen (DE); HACKEL, Kristin, 89564 Fleinheim (DE)
(86) Internationale Anmeldenummer: PCT/EP2015/059132
(87) Internationale Veröffentlichungsnummer: WO 2015/169637

(56) Entgegenhaltungen:
- EP-A1- 1 923 077
- EP-A1- 2 659 865
- EP-A1- 2 711 033
- WO-A1-2012/143665
- WO-A1-2012/143665
- WO-A1-2013/007732
- WO-A2-2012/082716
- US-A1- 2009 216 168
- US-A1- 2011 257 573
- US-A1- 2014 052 041
- US-A1- 2014 163 447
- STEPHEN THOMAS: "Wound Management and Dressings. Chapter 5: Foam Dressings" In: "The Pharmaceutical Press", 1990, London pages 35-42,

## Beschreibung

Die Erfindung betrifft eine beidseitig verwendbare Wundauflage zur Verwendung bei der Unterdrucktherapie von Wunden, mit einer ersten Lage aus einem ersten porösen Schaumstoff und mit einer zweiten Lage aus einem zweiten porösen Schaumstoff. Die erste und die zweite Lage sind unlösbar miteinander verbunden. Beide Lagen werden kanalartig und vollständig durch eine Vielzahl von durchgehenden Öffnungen durchdrungen. Der erste Schaumstoff und der zweite Schaumstoff unterscheiden sich hinsichtlich einer strukturellen Eigenschaft des Schaumstoffs.

Unterdrucktherapie von Wunden, im Fachbereich auch bezeichnet als topische negative Druckbehandlung (TNP = topical negative pressure) oder NPWT (negative pressure wound therapy), ist seit langem bekannt, findet aber insbesondere seit Mitte/Ende der 1990er Jahre in zunehmendem Maße Anwendung. Dabei wird üblicherweise ein Wundfüllmaterial in die Wunde eingelegt, das Wundgebiet mit einer Folie abgedeckt und mittels eines Drainageschlauchs und einer Vakuumpumpe ein Unterdruck im Wundraum erzeugt. Durch die Verwendung des Wundfüllmaterials wird der Druck gleichmäßig über die Wunde verteilt. Der Unterdruck bewirkt, insbesondere in der initialen Phase der Wundbehandlung, eine effektive Wundreinigung durch Abtransport von Wundexsudat. Als weitere Vorteile werden, insbesondere in der nachfolgenden Granulationsphase, Förderung der Bildung von Granulationsgewebe und Verminderung der Wundödembildung genannt. Die Unterdrucktherapie wird üblicherweise bis zum Wundverschluß durchgeführt. Der Wundverschluß gilt im Allgemeinen als erreicht, wenn über der vormaligen Wundstelle eine Schicht Epidermis vorhanden ist. Ein Wundverschluß kann durch Heilungsprozesse oder durch chirurgische Eingriffe erreicht werden. Unterdrucktherapie wird insbesondere eingesetzt bei akuten oder chronischen Wunden, beispielsweise bei Geschwüren (z.B. Druckgeschwüren, diabetischen, neuropathischen oder venös bedingten Geschwüren), traumatischen Wunden, therapieresistenten Wunden, infizierten Wunden, postoperativen Wunden, sondierten Fisteln sowie Hautlappen und Hauttransplantaten. Chronische Wunden sind durch eine verlangsamte oder fehlende Wundheilung gekennzeichnet.
Vorrichtungen zur Unterdrucktherapie von Wunden sind entsprechend im Stand der Technik bekannt. Geeignete Unterdrucktherapiegeräte und Zubehör werden beispielsweise in den Dokumenten WO2011018132, WO2011018133, WO2012156140, WO2012156174, WO2012163617 und WO2013159904 beschrieben. Die Dokumente WO2014023501 und WO2011076340 offenbaren geeignete Unterdruckanschlussmittel (Port) zum Herstellen einer Unterdruckverbindung mit dem Wundverband. In den Wundraum können beispielsweise die aus den Dokumenten WO2010072309, WO2012022484, WO2011072840, WO2012167942 oder WO2011003521 bekannten Wundauflagen eingebracht werden. Als besonders vorteilhaft in der Unterdruck-Wundbehandlung hat sich nach der Erfahrung des Anmelders eine Wundauflage aus einem offenzelligen Polyester-Polyurethanschaum, die in der EP2515812B1 (aus WO2012022485) näher beschrieben ist, erwiesen. Geräte zur Unterdrucktherapie von Wunden sind auch kommerziell erhältlich und umfassen kleine, tragbare Geräte, die den Patienten eine gewisse Mobilität ermöglichen bis hin zu stationär zu verwendenden Geräten, etwa in Langzeitpflegeeinrichtungen. Auch für die Unterdrucktherapie geeignetes Zubehör wie beispielsweise Wundauflagen, weitere Verbandkomponenten oder Anschlussmittel sind kommerziell erhältlich.

Die Unterdrucktherapie kann zur Behandlung einer Vielzahl unterschiedlicher Wundtypen eingesetzt werden, beispielsweise Druckwunden (Dekubitus), Geschwüren (Ulcus), Brandwunden oder Wunden infolge traumatischer Einwirkung. Insbesondere findet die Unterdrucktherapie Anwendung bei der Behandlung schlecht heilender und/oder infizierter Wunden.

In der Literatur werden drei wesentliche Heilungsphasen einer Wunde, insbesondere bei Wunden mit Gewebeverlust, beschrieben. Hierzu gehört die Entzündungs-(inflammatorische) oder exsudative Phase zur Blutstillung und Wundreinigung (Phase 1, Reinigungsphase), die proliferative Phase zum Aufbau von Granulationsgewebe (Phase 2, Granulationsphase) und die Differenzierungsphase zur Epithelisierung und Narbenbildung (Phase 3, Epithelisierungsphase).

Die inflammatorische Phase tritt üblicherweise direkt nach der Wundbildung auf. Sie ist gekennzeichnet durch Gefäßkonstriktion, Aktivierung der Gerinnungskaskade und komplexe immunologische Abläufe. Durch die Freisetzung chemoattraktiver Substanzen (z.B. Chemokine) kann eine lokale Entzündungsreaktion hervorgerufen werden. Es gibt vier wesentliche Entzündungszeichen: Ödem, Exsudation, Rötung und Schmerz. Die umliegenden Gefäße können sich erweitern, und Entzündungszellen wandern in den Entzündungsort ein. Diese können Mikroorganismen und Gewebsnekrosen beseitigen. Dadurch kann eine Reinigung der Wunde erfolgen.

Bei der darauf folgenden Proliferations- oder Granulationsphase erfolgt der Aufbau von neuem Gewebe mit Gefäßeinsprossung und Defektauffüllung durch Granulationsgewebe. Dies ist die Grundvoraussetzung für die spätere Epithelisierung. Fibroblasten aus dem umliegenden Gewebe können in das Fibrinnetz migrieren und es als provisorische Matrix nutzen. Es beginnt der Aufbau von Kollagenfasern. Das provisorische Fibringerüst wird durch das Granulationsgewebe ersetzt. Die gleichzeitig stattfindende Angiogenese versorgt das Granulationsgewebe mit einsprossenden Kapillaren und Gefäßen.
Mit der Differenzierungs- oder Umbauphase beginnt üblicherweise die Ausreifung der kollagenen Fasern. Die Wunde kontrahiert sich durch die Umwandlung von Fibroblasten in Fibrozyten sowie Myofibroblasten. Dadurch schrumpft das Narbengewebe und es kommt zu einer Verkleinerung der Wunde. Die Epithelisierung vom Wundrand her bringt die Wundheilung zum Abschluss.

Die oben angesprochenen Phasen der Wundheilung können in den unterschiedlichen Bereichen einer einzigen Wunde auch gleichzeitig nebeneinander vorliegen.

Die derzeit in der Unterdrucktherapie am häufigsten verwendeten Wundauflagen umfassen einen offenzelligen Polymerschaum aus Polyurethan oder Polyvinylalkohol, siehe hierzu beispielsweise die vorstehend bereits erwähnte EP2515812B1. Derartige Wundauflagen können für eine Vielzahl typischerweise auftretender Wundsituationen eine ausreichende Versorgung gewährleisten. Jedoch könnten im Rahmen einer "phasengerechten Wundbehandlung" zusätzliche verbesserte Therapieerfolge erzielt werden, wenn die verwendete Wundauflage auf die jeweilige Phase der Wundheilung abgestimmt ist. Ansätze zur phasengerechten Wundbehandlung in der Unterdrucktherapie sind beispielsweise bereits in der WO2014048514 beschrieben, wobei die dort offenbarten Therapieverfahren auf einen ersten Therapieabschnitt unter Verwendung der Unterdrucktherapie und einen nachfolgenden Therapieabschnitt ohne Anwendung von Unterdruck, jedoch unter Verwendung eines Saugkörpers, welcher ein quellfähiges Polymer umfasst, gerichtet sind.

Bei einer Schaumstoffwundauflage scheint nach bisher vorliegenden Erfahrungen beispielsweise die Porengröße, die chemische Schaumstoff-Zusammensetzung oder auch die Weichheit des verwendeten Materials einen deutlichen Effekt auf den Anwendungserfolg zu haben. Jedoch können sich hierbei mit Bezug auf die genannten Wundheilungsphasen abweichende Anforderungen ergeben. Insbesondere zu Beginn der Wundbehandlung, also in der dann typischerweise vorliegenden Reinigungsphase, können erhebliche Mengen an infektiösem Material und Debris vorliegen, welche bei Verwendung einer Schaumstoffwundauflage die Poren des Füllmaterials zusetzen können. In der Folge kann Exsudatstau, Mazeration, Infektion oder sogar Sepsis auftreten. In der Reinigungsphase könnte deshalb bei bestimmten Wundsituationen beispielsweise ein besonders durchlässiger Wundschaum wünschenswert sein. In anderen Fällen könnte in der ersten Heilungsphase ein besonders weicher Schaum wünschenswert sein, beispielsweise bei der Unterdruckbehandlung empfindlicher Gewebestellen. In den nachfolgenden Behandlungsphasen, insbesondere in der Granulationsphase und in der Differenzierungs- oder Umbauphase hingegen können sich dann veränderte Anforderungen an eine Wundauflage ergeben.

Der vorliegenden Erfindung liegt die Aufgabe zugrunde, eine Unterdruck-Wundtherapie bereit zu stellen, welche den physiologischen Zustand der Wunde positiv beeinflusst, insbesondere positiver beeinflusst als dies mit der herkömmlichen Unterdrucktherapie möglich ist.

Ferner soll eine Wundtherapie bereitgestellt werden, welche den Patienten möglichst wenig beeinträchtigt. Zudem war es eine Aufgabe, die Wundtherapie hinsichtlich ihrer Kosten zu verbessern, ohne jedoch die klinische Effizienz der Wundtherapie zu beeinträchtigen oder aber dabei die klinische Effizienz sogar zu erhöhen. Weiterhin war es Aufgabe, dem Anwender möglichst einfach zu verwendende Mittel für die Unterdrucktherapie zur Verfügung zu stellen.

Es wurde eine verbesserte Unterdruck-Wundauflage gefunden, die für eine Vielzahl von Wundsituation mit Erfolg eingesetzt werden kann. Die gemäß Anspruch 1 vorgeschlagene Wundauflage ermöglicht die Behandlung einer Vielzahl von Wundsituationen. Insbesondere kann mit nur einer einzigen Wundauflage eine phasengerechte Wundbehandlung durchgeführt werden. Insbesondere wurde unerwartet entdeckt, dass durch die mittels der erfindungsgemäßen Wundauflage durchgeführte phasengerechte Wundbehandlung zahlreiche Wundparameter eine unerwartete Verbesserung zeigen und somit ein unerwartet guter Heilungsverlauf der Wunde zu beobachten ist.

Erfindungsgemäß vorgeschlagen wird eine Wundauflage zur Verwendung bei der Unterdrucktherapie von Wunden, umfassend eine erste Lage aus einem ersten porösen Schaumstoff und eine zweite Lage aus einem zweiten porösen Schaumstoff. Die zweite Lage ist mit der ersten Lage unlösbar verbunden, beispielsweise durch Verkleben oder Hitzelaminieren. Die erste Lage und die zweite Lage stehen also in unmittelbarer und untrennbarer Verbindung zueinander. Beide Lagen werden kanalartig und vollständig von einer Vielzahl durchgehenden Öffnungen durchdrungen, welche vorzugsweise mit Bezug auf die flächige Ausdehnung der Lagen im Wesentlichen vertikal angeordnet sind. Vorzugsweise werden die Öffnungen, welche beide Lagen kanalartig und vollständig durchdringen, erst nach dem Herstellen der unlösbaren Verbindung von erster und zweiter Lage eingebracht.

Die durchgehenden Öffnungen weisen vorzugsweise einen Durchmesser von mindestens 1 mm und höchstens 15 mm, besonders bevorzugt von mindestens 2 mm und höchstens 10 mm, insbesondere von mindestens 4 mm und höchstens 6 mm auf. Dabei können beispielsweise 3 bis 200, vorzugsweise 5 bis 100 und insbesondere 10 bis 50 Öffnungen auf 100 cm² Oberfläche der Wundauflage vorhanden sein. Die Wundauflage ist erfindungsgemäß weiter dadurch gekennzeichnet, dass sich der erste Schaumstoff hinsichtlich einer der Eigenschaft des Schaumstoffs umfassend die Zellzahl, die Rohdichte, die Stauchhärte oder die Bruchdehnung von dem zweiten Schaumstoff unterscheidet.
Die den Schaumstoff unterscheidende Eigenschaft umfasst demnach die Zellzahl, gemessen in Zellen pro Inch, die Rohdichte, gemessen gemäß DIN EN ISO 845 (Ausgabe Oktober 2009; vor der Bestimmung der Rohdichte erfolgt 72 Stunden Konditionierung bei Normklima, 23°C, 50 % relative Feuchte; die Bestimmung der Rohdichte erfolgt gleichfalls bei Normklima), die Stauchhärte, gemessen nach ISO 3386-1 (wie unten näher dargestellt) oder die Bruchdehnung, gemessen nach DIN 53571 (Ausgabe Januar 1986; Verfahren 1, Probekörper A; vor der Bestimmung der Bruchdehnung erfolgt 72 Stunden Konditionierung bei Normklima, 23°C, 50 % relative Feuchte; die Bestimmung der Bruchdehnung erfolgt gleichfalls bei Normklima). Mit dem Ausdruck "Stauchhärte" ist im vorliegenden Dokument stets der Druckspannungswert CV40 gemeint, welcher die Kraft bezeichnet, die zu einer Verformung des Materials von 40 % erforderlich ist. Die Stauchhärte wird nach DIN ISO 3386-1, Ausgabe September 2010, bestimmt. Vor der Bestimmung der Stauchhärte erfolgt 72 Stunden Konditionierung bei Normklima, 23°C, 50 % relative Feuchte. Die Bestimmung der Stauchhärte erfolgt dann gleichfalls bei Normklima. Zur Bestimmung der Stauchhärte wird vorzugsweise ein Probenkörper mit einer Dicke von 30 mm und einer Grundfläche von 100 x 100 mm verwendet.
Sofern im vorliegenden Dokument von den Schaumstoff-Eigenschaften Zellzahl, Bruchdehnung, Stauchhärte oder Rohdichte die Rede ist, sind mit diesen Parametern stets die Eigenschaften des Ausgangsmaterials (Schaumstoff) gemeint. Die Parameter werden deshalb an einem einlagigen Schaumstoff-Probenstück bestimmt, welches keine Öffnungen aufweist. Die bei der erfindungsgemäßen Wundauflage vorgesehenen, beide Lagen kanalartig und vollständig durchdringen Öffnungen, sind bei dem zur Bestimmung der Schaumstoff-Eigenschaften heranzuziehenden Material also noch nicht vorhanden.
Die Wundauflage kann bei der Anwendung in der Unterdrucktherapie nach Wahl des Anwenders sowohl mit ihrer ersten Lage zum Wundgrund hin zeigend als auch mit ihrer zweiten Lage zum Wundgrund hin zeigend in die Wunde eingebracht werden. Der Anwender kann also anhand der Wundsituation entscheiden, welche der beiden Seiten er zum Wundgrund hin zeigend auflegt. Gemäß eines zur Verdeutlichung der Erfindung angeführten Ausführungsbeispiels umfasst die erste Lage einen ersten Schaumstoff, welcher eine Zellzahl von 8 Poren pro Inch aufweist, während die zweite Lage einen zweiten Schaumstoff umfasst, der eine Zellzahl von 50 Poren pro Inch aufweist. Gemäß dieser hier nur beispielhaft erwähnten Ausführungsform wäre die erste Lage insbesondere zur Unterdruck-Wundbehandlung in der Reinigungsphase geeignet, während die zweite Lage insbesondere zur Unterdruck-Wundbehandlung in der Granulationsphase oder in einer späteren Wundheilungsphase (insbesondere Differenzierung- bzw. Umbauphase) indiziert wäre. Entsprechend würde der Anwender hier zur Behandlung einer Wunde, welche sich in der Reinigungsphase befindet, die erste Lage zum Wundgrund hin zeigend aufbringen. Dagegen würde der Anwender zur Behandlung einer Wunde, welche sich in der Granulationsphase oder in einer späteren Wundheilungsphase befindet, die zweite Lage zum Wundgrund hin zeigend aufbringen.

Die Erfindung schlägt somit eine beidseitig verwendbare Wundauflage für die Unterdrucktherapie vor, wobei jede der Seiten für eine bestimmte Wundheilungsphase besonders vorteilhaft eingesetzt werden kann. Hierbei hat es sich als erfindungswesentlich herausgestellt, dass die mindestens zwei Lagen umfassende Wundauflage eine Vielzahl durchgehender Öffnungen aufweist, wobei die Öffnungen vorzugsweise vertikal angeordnet sind. Es hat sich gezeigt, dass die Öffnungen wesentlich sind, um eine zuverlässige Exsudatableitung und eine gleichmäßige Verteilung des Unterdrucks bei den typischerweise auftretenden Wundsituationen und über alle Phasen der Wundbehandlung hinweg dauerhaft zu gewährleisten.

Die erfindungsgemäße Wundauflage kann ohne weiteres durch den Anwender zugeschnitten werden, um eine Anpassung an die jeweilige Größe und den jeweiligen Umfang der Wunde zu erreichen. Ein besonderer Vorteil besteht darin, dass auf unterschiedliche Bereiche einer einzigen Wunde, welche sich in unterschiedlichen Wundheilungsphasen befinden, jeweils die erste oder die zweite Lage appliziert werden kann. Hierzu müssen aus der Wundauflage geeignete Stücke herausgeschnitten werden, die dann jeweils entweder mit ihrer ersten Lage oder mit ihrer zweiten Lage zum Wundgrundhin zeigend aufgelegt werden. Es können also beispielsweise auf Bereiche der Wunde, welche sich nach Einschätzung des Anwenders in der Reinigungsphase befinden, Wundauflagen-Stücke mit ihrer ersten Lage zum Wundgrund hin zeigend appliziert werden. Gleichzeitig können auf daneben vorhandene Bereiche der Wunde, welche sich nach Einschätzung des Anwenders in der Granulationsphase befinden, Wundauflagen-Stücke mit ihrer zweiten Lage zum Wundgrund hin zeigend aufgelegt werden.

Vorzugsweise weist sowohl die erste Lage als auch die zweite Lage eine Dicke von mindestens 5,0 mm und höchstens 30,0 mm, insbesondere mindestens 6,0 mm und höchstens 20,0 mm, auf. Eine derartige Wundauflage ist für eine Vielzahl in der Praxis auftretender Wundbehandlungen geeignet. Die erste Lage und die zweite Lage kann jeweils eine unterschiedliche Dicke aufweisen. Die Dicke wird gemäß ISO 1923 (zweite Ausgabe -1981-09-01), wie in Abschnitt 4.4 der Vorschrift angegeben, mit einem Mikrometer bestimmt, wobei auf 0,1 mm aufgerundet wird.

Gemäß einer vorteilhaften Ausführungsform weist eine zur Behandlung einer Wunde in der Reinigungsphase vorgesehene erste Lage eine Dicke (gemessen gemäß ISO 1923) von mindestens 15,0 mm und höchstens 30,0 mm auf, während eine zur Behandlung einer Wunde in der Granulationsphase vorgesehene zweite Lage eine Dicke von mindestens 5,0 mm und höchstens 20,0 mm aufweist.
Gegebenenfalls können bei der Behandlung besonders tiefer Wunden mehrere Schichten der erfindungsgemäßen Wundauflage (welche jeweils eine erste und eine zweite Lage umfassen) übereinander angeordnet werden. Hierbei hat es sich als vorteilhaft erwiesen, wenn bei der dann erforderlichen Stapelung jeweils eine erste und eine zweite Lage aneinander grenzend aufgebracht werden.

Gemäß einer besonders vorteilhaften Ausführungsform besteht die Wundauflage ausschließlich aus der ersten Lage und der zweiten Lage. Eine derartige Wundauflage kann besonders einfach durch Zuschneiden, beispielsweise mittels einer sterilen Schere, an die Wundform angepasst werden. Jedoch können bei der Wundbehandlung zusätzliche Verbandschichten eingesetzt werden, beispielsweise eine Wundkontaktschicht aus einer Folie mit atraumatischen Eigenschaften. Diese zusätzlichen Verbandschichten sind gemäß der hier vorgestellten vorteilhaften Ausführungsform nicht mit der erfindungsgemäßen Wundauflage unlösbar verbunden.

Die in der Wundauflage vorgesehenen Öffnungen können beispielsweise einen kreisrunden, einen ovalen, einen rechteckigen, einen quadratischen oder einen sternförmigen Querschnitt aufweisen. Die Öffnungen werden vorzugsweise in die Wundauflage eingebracht, nachdem die erste und die zweite Lage miteinander verbunden worden sind. Das Einbringen der Öffnungen kann mit üblichen Verfahren, beispielsweise durch Bohren oder Stanzen, erfolgen.

Es ist erforderlich, dass sowohl die erste Lage als auch die zweite Lage aus einem Schaumstoff besteht, welcher zum direkten Kontakt mit einem Wundgrund geeignet ist. Es muss sich also jeweils um einen für die Wundbehandlung geeigneten Schaumstoff handeln, beispielsweise um einen Schaumstoff aus Polyurethan-Ester, Polyurethan-Ether oder Polyvinylalkohol. Ein für die Unterdrucktherapie besonders geeigneter Schaumstoff aus Polyurethan-Ester wird in der bereits erwähnten EP2515812B1 beschrieben.

Gemäß einer bevorzugten Ausführungsform der Wundauflage umfasst der erste Schaumstoff und/oder der zweite Schaumstoff ein Polymer oder eine Polymermischung ausgewählt aus Polyurethan-Ester, Polyurethan-Ether, Polyvinylalkohol, Polyurethan-Polyharnstoff-Copolymer, Polyorganosiloxan, Polyurethan-Silicon-Copolymer, Polylactide, Chitosan oder Kollagen. Erster und zweiter Schaumstoff können dieselbe chemische Zusammensetzung oder eine unterschiedliche chemische Zusammensetzung aufweisen.

Gemäß einer weiteren bevorzugten Ausführungsform der Wundauflage unterscheiden sich die erste und die zweite Lage hinsichtlich der chemischen Zusammensetzung von erstem und zweitem Schaumstoff. Der erste Schaumstoff umfasst bei dieser Ausführungsform beispielsweise ein erstes Polymer oder eine erste Polymermischung ausgewählt aus Polyurethan-Ester, Polyurethan-Ether, Polyvinylalkohol, Polyurethan-Polyharnstoff-Copolymer, Polyorganosiloxan, Polyurethan-Silicon-Copolymer, Polylactide, Chitosan oder Kollagen. Der zweite Schaumstoff umfasst bei dieser Ausführungsform dann beispielsweise ein zweites Polymer oder eine zweite Polymermischung ausgewählt aus Polyurethan-Ester, Polyurethan-Ether, Polyvinylalkohol, Polyurethan-Polyharnstoff-Copolymer, Polyorganosiloxan, Polyurethan-Silicon-Copolymer, Polylactide, Chitosan oder Kollagen, mit der Maßgabe, dass sich das erste Polymer oder - falls zutreffend - die erste Polymermischung von dem zweiten Polymer oder - falls zutreffend - von der zweiten Polymermischung unterscheidet.

Gemäß einer gleichfalls bevorzugten Ausführungsform der Wundauflage unterscheiden sich die erste und die zweite Lage sowohl hinsichtlich der chemischen Zusammensetzung von erstem und zweitem Schaumstoff als auch in einer der Eigenschaften von erstem und zweitem Schaumstoff umfassend die Zellzahl, die Rohdichte, die Stauchhärte oder die Bruchdehnung.

Erfindungsgemäß handelt es sich bei dem ersten und dem zweiten Schaumstoff um einen offenzelligen Schaumstoff, insbesondere um einen retikulierten Schaumstoff.

In einer bevorzugten Ausführungsform ist der erste und/oder der zweite Schaumstoff ein offenzelliger Polyurethanschaumstoff, der erhältlich ist durch Umsetzung einer

Mischung, umfassend die Komponenten (i) Polyisocyanat, (ii) Polyol, bevorzugt Polyesterpolyol, (iii) Treibmittel, und (iv) Katalysator.

Als Polyisocyanate (i-PUR) können allgemein bekannte aliphatische, cycloaliphatische und/oder insbesondere aromatische Polyisocyanate eingesetzt werden. Zur Herstellung der Polyurethane eignen sich beispielsweise Diphenylmethandiisocyanat (MDI), hier insbesondere 4,4'- Diphenylmethandiisocyanat (4,4'-MDI), Mischungen aus monomeren Diphenylmethan-diisocyanaten und höherkernigen Homologen des Diphenylmethandiisocyanats (PMDI), Tetramethylendiisocyanat (TMDI), Hexamethylendiisocyanat (HDI), Toluylendiisocyanat (TDI) oder Mischungen daraus.

Als gegenüber Isocyanaten reaktive Verbindungen (ii-PUR) werden üblicherweise Polyole wie Polyetherole und/oder Polyesterole verwendet. Bevorzugt werden Polyesterpolyole in der Komponente (ii-PUR) verwendet. Die verwendeten Polyesterole (ii-PUR) werden im allgemeinen durch Kondensation von mehrfunktionellen Alkoholen, vorzugsweise Diolen, mit 2 bis 12 Kohlenstoffatomen, vorzugsweise 2 bis 6 Kohlenstoffatomen, mit mehrfunktionellen Carbonsäuren mit 2 bis 12 Kohlenstoffatomen, bevorzugt 4 bis 8 Kohlenstoffatomen hergestellt. Beispiele für geeignete Säuren sind Bernsteinsäure, Glutarsäure, Adipinsäure, Phthalsäure, Isophthalsäure, und/oder Terephthalsäure und Gemische hiervon. Adipinsäure ist besonders bevorzugt. Beispiele für geeignete zwei- und mehrwertige Alkohole sind Ethandiol, Diethylenglykol, 1,4-Butandiol, 1,5-Pentandiol, und/oder 1,6-Hexandiol und Gemische hiervon. 1,4-Butandiol ist besonders bevorzugt. Die Verbindungen (ii-PUR) können in Mischung mit Kettenverlängerungs- und/oder Vernetzungsmitteln verwendet werden.

Als Katalysatoren (iv-PUR) können Verbindungen eingesetzt werden, welche die Reaktion der Komponente (i-PUR) mit der Komponente (ii-PUR) beschleunigen. In Frage kommen beispielsweise tertiäre Amine und/oder organische Metallverbindungen, insbesondere Zinnverbindungen. Beispielsweise können als Katalysatoren folgende Verbindungen eingesetzt werden: Triethylendiamin, Aminoalkyl- und/oder Aminophenylimidazole und/oder Zinn-(II)salze von organischen Carbonsäuren. Katalysatoren werden im Allgemeinen in einer Menge von 0,1 bis 5 Gew.-% bezogen auf das Gewicht der Komponente (ii-PUR) eingesetzt.

Als Treibmittel (iii-PUR) können allgemein bekannte chemisch oder physikalisch wirkende Verbindungen eingesetzt werden. Als physikalisch wirkendes Treibmittel kann bevorzugt Wasser eingesetzt werden, welches durch Reaktion mit den Isocyanatgruppen Kohlendioxid bildet. Beispiele für physikalische Treibmittel sind (cyclo)aliphatische Kohlenwasserstoffe, vorzugsweise solche mit 4 bis 8, besonders bevorzugt 4 bis 6 und insbesondere 5 Kohlenstoffatomen, teilhalogenierte Kohlenwasserstoffe oder Ether, Ketone oder Acetate. Die Menge der eingesetzten Treibmittel richtet sich nach der angestrebten Dichte der Schaumstoffe. Die unterschiedlichen Treibmittel können einzeln oder in beliebigen Mischungen untereinander zum Einsatz kommen. Besonders bevorzugt wird nur Wasser als Treibmittel eingesetzt, im allgemeinen in einer Menge von 0,1 bis 5 Gew.-%, insbesondere von 2,5 bis 4 Gew.-%, bezogen auf das Gewicht der Komponente (ii-PUR). Physikalische Treibmittel werden bevorzugt in einer Menge von < 0,5 Gew.-%, bezogen auf das Gewicht der Komponente (ii-PUR), eingesetzt.

Die Umsetzung erfolgt gegebenenfalls in Anwesenheit von (v-PUR) Hilfs- und/oder Zusatzstoffen, wie z. B. Füllstoffen, Zellreglern, Zellöffnern, oberflächenaktiven Verbindungen und/oder Stabilisatoren gegen oxidativen, thermischen oder mikrobiellen Abbau oder Alterung.

Bevorzugt ist der offenzellige Polyurethanschaumstoff erhältlich durch Umsetzung einer Mischung, die die folgenden Komponenten umfasst oder aus diesen besteht:
(i) Polyisocyanat, ausgewählt aus MDI, PMDI, TDI und/oder HDI,
(ii) Polyesterpolyol, wobei das Polyesterpolyol bevorzugt erhältlich ist durch Umsetzung einer Dicarbonsäure mit 4 bis 8 Kohlenstoffatomen mit einen Dialkohol mit 2 bis 6 Kohlenstoffatomen und/oder bevorzugt ein gewichtsmittleres Molekulargewicht von 500 bis 4000 g/mol aufweist,
(iii) Treibmittel, und
(iv) Katalysator.

Als Schaumstoffe werden üblicherweise Werkstoffe mit über die gesamte Masse verteilten Zellen (offen, geschlossen oder beides) verstanden. Solche Werkstoffe weisen üblicherweise somit eine Rohdichte (gemäß DIN EN ISO 845) auf, die niedriger ist als die Dichte der Gerüstsubstanz. Eine Zelle ist der bei der Herstellung von Schaumstoffen gebildete einzelne Hohlraum, der von Zellwänden und/oder Zellstegen teilweise oder vollständig umschlossen ist. Eine geschlossene Zelle ist üblicherweise eine Zelle, die vollständig von ihren Wänden umschlossen ist und daher nicht mit anderen Zellen über die Gasphase in Verbindung steht. Eine offene Zelle ist üblicherweise eine Zelle, die über die Gasphase mit anderen Zellen in Verbindung steht. Im Rahmen dieser Anmeldung bedeutet der Begriff offenzellig, dass im Schaumstoff mindestens 60 % offene Zellen, bevorzugt mindestens 90 % offene Zellen, mehr bevorzugt mindestens 98 % offene Zellen, insbesondere im Wesentlichen 100 % offene Zellen, bezogen auf die Gesamtzahl an Zellen, vorhanden sind. Die Offenzelligkeit des Schaumstoffs wird üblicherweise nach ASTM D 2856-87, Verfahren B) bestimmt. Unter Zellwand wird üblicherweise die die Zelle umschließende Wand verstanden. Die Zellwand kann auch als Zellmembran bezeichnet werden. Als Zellsteg wird üblicherweise der Bereich der Zellwand verstanden, der mehr als zwei Zellen voneinander trennt. Zellstege weisen bevorzugt mindestens das 1,5-fache der Dicke, mehr bevorzugt mindestens das 2-fache der Dicke, des übrigen Bereichs der Zellwand auf. Bei dem offenzelligen Schaumstoff kann es sich um einen retikulierten oder um einen nicht-retikulierten Schaumstoff handeln. Unter einem retikulierten Schaumstoff wird ein Schaumstoff verstanden, der im Wesentlichen nur Zellstege aufweist. Bei einem retikulierten Schaumstoff sind die Zellwände somit im Wesentlichen entfernt. Die Retikulierung wird üblicherweise in einer Druckkammer, z.B. einer Stahlkammer durchgeführt. Nach Einbringen des Schaumstoffs in die Stahlkammer wird die Luft abgesaugt (bevorzugt zu 50 bis 100 Gew.-%, mehr bevorzugt zu 70 bis 99 Gew.-%) und durch ein Brenngasgemisch, bevorzugt durch ein Gemisch enthaltend Wasserstoff und Sauerstoff, insbesondere im molaren Verhältnis von 2 : 1 ersetzt. Bei der Zündung des Gasgemisches zerreißen die Zellhäute durch die entstehende Hitze- und Druckwelle.
Gegebenenfalls erfolgt auch ein zumindest teilweises Aufschmelzen der Zellstege, so dass diese verstärkt werden.

Es hat sich ferner gezeigt, dass vorstehend genannte Aufgaben unerwartet vorteilhaft gelöst werden können, wenn der erste und/oder der zweite Schaumstoff eine spezielle Luftdurchlässigkeit aufweist. In einer bevorzugten Ausführungsform weist der Schaumstoff eine Luftdurchlässigkeit von 1000 bis 8000 l/(m²sec), mehr bevorzugt von 1500 bis 6000 l/(m²sec), noch mehr bevorzugt von 2000 bis 5000 l/(m²sec), besonders bevorzugt von 2300 bis 4000 l/(m²sec) und insbesondere von 2400 bis 3300 l/(m²sec), gemessen gemäß DIN EN ISO 9237 (20 mm Prüfdicke, 20 cm² Prüffläche, 200 Pa Differenzdruck) auf.
Hierbei hat es sich als besonders vorteilhaft erwiesen, wenn die Luftdurchlässigkeit des ersten Schaumstoffs um mindestens den Faktor 1,1 und um höchstens den Faktor 8,0 höher ist als die Luftdurchlässigkeit des zweiten Schaumstoffs (d.h. Luftdurchlässigkeit des zweiten Schaumstoffs multipliziert mit 1,1 ergibt die minimale Luftdurchlässigkeit des ersten Schaumstoffs; Luftdurchlässigkeit des zweiten Schaumstoffs multipliziert mit 8,0 ergibt die maximale Luftdurchlässigkeit des ersten Schaumstoffs, wobei die maximale Luftdurchlässigkeit des ersten Schaumstoffs den Wert 8000 l/(m²sec) nicht übersteigt). Besonders bevorzugt ist die Luftdurchlässigkeit des ersten Schaumstoffs um mindestens den Faktor 1,5 und um höchstens den Faktor 8,0 höher als die Luftdurchlässigkeit des zweiten Schaumstoffs, insbesondere ist die Luftdurchlässigkeit des ersten Schaumstoffs um mindestens den Faktor 2,0 und um höchstens den Faktor 5,0 höher als die Luftdurchlässigkeit des zweiten Schaumstoffs.

Ferner weist der erste und/oder der zweite Schaumstoff bevorzugt eine Bruchdehnung von 100 % bis 400 %, mehr bevorzugt von 120 % bis 300 %, noch mehr bevorzugt von 150 % bis 200 %, gemessen gemäß DIN 53571, auf. Vorzugsweise ist die Bruchdehnung des ersten Schaumstoffs, gemessen gemäß DIN 53571, um mindestens den Faktor 1,05 und um höchstens den Faktor 3,50 größer als die Bruchdehnung des zweiten Schaumstoffs (d.h. Bruchdehnung des zweiten Schaumstoffs multipliziert mit 1,05 ergibt die minimal Bruchdehnung des ersten Schaumstoffs; Bruchdehnung des zweiten Schaumstoffs multipliziert mit 3,50 ergibt die maximale Bruchdehnung des ersten Schaumstoffs, wobei die maximale Bruchdehnung den Wert 350 % nicht übersteigt). Besonders bevorzugt ist die Bruchdehnung des ersten Schaumstoffs um mindestens den Faktor 1,10 und um höchstens den Faktor 2,50 größer als die Bruchdehnung des zweiten Schaumstoffs, insbesondere ist die Bruchdehnung des ersten Schaumstoffs um mindestens den Faktor 1,20 und um höchstens den Faktor 2,00 größer als die Bruchdehnung des zweiten Schaumstoffs.

Gemäß einer weiteren bevorzugten Ausführungsform der Erfindung weist der erste und/oder der zweite Schaumstoff eine Zellzahl (= Anzahl der Poren entlang einer Geraden pro laufendem Inch) von mindestens 5 und höchstens 400 Zellen pro Inch auf. Die Zellzahl wird bevorzugt mikroskopisch bestimmt.
Hierbei ist es besonders vorteilhaft, wenn die erste Lage eine Zellzahl zwischen 5 und 200 Zellen pro Inch, vorzugsweise eine Zellzahl zwischen 6 und 100 Zellen pro Inch, besonders bevorzugt eine Zellzahl zwischen 8 und 30 Zellen pro Inch aufweist. Gleichzeitig sollte die zweite Lage vorzugsweise zwischen 10 und 400 Zellen pro Inch, besonders bevorzugt zwischen 15 und 200 Zellen pro Inch und insbesondere zwischen 40 und 60 Zellen pro Inch aufweisen.
Gemäß einer alternativen, gleichfalls sehr vorteilhaften Ausführungsform, beträgt die Zellzahl des ersten und/oder der zweiten Schaumstoff mindestens 10 und höchstens 200 Zellen pro Inch, wobei die Zellzahl des zweiten Schaumstoffs um mindestens den Faktor 1,1 und höchstens den Faktor 20,0 größer als die Zellzahl des ersten Schaumstoffs ist (d.h. Zellzahl des ersten Schaumstoffs multipliziert mit 1,1 ergibt die Mindest-Zellzahl des zweiten Schaumstoffs; Zellzahl des ersten Schaumstoffs multipliziert mit 20,0 ergibt die maximale Zellzahl des zweiten Schaumstoffs, wobei der zweite Schaumstoff eine Zellzahl von höchstens 200 Zellen pro Inch aufweist). Vorzugsweise ist die Zellzahl des zweiten Schaumstoffs um mindestens den Faktor 1,2 , insbesondere um mindestens den Faktor 1,5 größer als die Zellzahl des ersten Schaumstoffs. Der erste Schaumstoff ist bei dieser Ausführungsform besonders zum Inkontaktbringen mit einer Wundoberfläche, welche sich in der Reinigungsphase befindet, geeignet. Gleichzeitig ist der zweite Schaumstoff bei dieser Ausführungsform besonders zum Inkontaktbringen mit einer Wundoberfläche, welche sich in der Granulationsphase befindet, geeignet.

Gemäß einer weiteren vorteilhaften Ausführungsform ist vorgesehen, dass die Rohdichte des ersten und/oder des zweiten Schaumstoffs zwischen 10 und 350 kg/m³ aufweisen sollte, gemessen gemäß DIN EN ISO 845. Besonders bevorzugt beträgt die Rohdichte 15 bis 65 kg/m³, insbesondere 20 bis 45 kg/m³. Weiterhin ist es vorteilhaft, wenn sich die Rohdichte des ersten Schaumstoffs um mindestens den Faktor 1,1 und höchstens um den Faktor 20,0 oder noch vorteilhafter um mindestens den Faktor 1,2 und höchstens um den Faktor 15,0 von der Rohdichte des zweiten Schaumstoffs unterscheidet. Möglich ist hierbei, dass der erste Schaumstoff eine höhere Rohdichte im Vergleich zum zweiten Schaumstoff aufweist. Alternativ kann der zweite Schaumstoff eine höhere Rohdichte im Vergleich zum ersten Schaumstoff aufweisen.

Die Stauchhärte des für die erste oder für die zweite Lage vorgesehenen ersten oder zweiten Schaumstoffs beträgt vorzugsweise mindestens 1,5 kPa und höchstens 22,5 kPa, gemessen nach ISO 3386-1. Hierbei ist es vorteilhaft, wenn sich die Stauchhärte des ersten Schaumstoffs um mindestens den Faktor 1,1 und um höchstens den Faktor 15,0 oder noch vorteilhafter um mindestens den Faktor 1,2 und um höchstens den Faktor 10,0 von der Stauchhärte des zweiten Schaumstoffs unterscheidet. Gemäß einer besonders vorteilhaften Ausführungsform ist die Stauchhärte des ersten Schaumstoffs um mindestens den Faktor 1,1 und um höchstens den Faktor 15,0 höher als die Stauchhärte des zweiten Schaumstoffs (d.h. Stauchhärte des zweiten Schaumstoffs multipliziert mit 1,1 ergibt die Mindest-Stauchhärte des ersten Schaumstoffs; Stauchhärte des zweiten Schaumstoffs multipliziert mit 15,0 ergibt die maximale Stauchhärte des ersten Schaumstoffs, wobei der erste Schaumstoff eine maximale Stauchhärte von höchstens 22,5 kPa aufweist). Insbesondere ist die Stauchhärte des ersten Schaumstoffs um mindestens den Faktor 1,5 und um höchstens den Faktor 10,0 höher als die Stauchhärte des zweiten Schaumstoffs. Der erste Schaumstoff ist bei dieser Ausführungsform speziell zum Inkontaktbringen mit einer Wundoberfläche, welche sich in der Reinigungsphase befindet, geeignet. Gleichzeitig ist der zweite Schaumstoff bei dieser Ausführungsform speziell zum Inkontaktbringen mit einer Wundoberfläche, welche sich in der Granulationsphase befindet, geeignet.

Der von der Wundauflage umfasste erste und/oder der zweite Schaumstoff kann mit einem Zusatz und/oder Hilfsstoff beschichtet oder imprägniert sein. Bei dem Zusatz oder der Imprägnierung kann es sich beispielsweise um eine die Wundheilung fördernde Salbengrundlage handeln, wobei die Salbengrundlage vorzugsweise zusätzlich einen Quellstoff umfasst. Exemplarisch wird auf die WO2012/167943 verwiesen, welche im Rahmen der Erfindung geeignete Salbengrundlagen beschreibt. Eine im Rahmen der Erfindung besonders geeignete Salbengrundlage, welche zusätzlich einen Quellstoff umfasst, ist in Beispiel 1 der Patentanmeldung WO2012/167943 beschrieben.
Der von der Wundauflage umfasste erste und/oder der zweite Schaumstoff kann alternativ oder zusätzlich zu der vorstehend beschriebenen Salbengrundlage eine antimikrobiell wirksame Substanz umfassen, beispielsweise Polyhexanid. Bei der antimikrobiell wirksamen Substanz kann es sich auch um ein Antibiotikum handeln, beispielsweise um Oxytetracyclin oder um eine Kombination von Bacitracin und Neomycin. Gegebenenfalls kann der Zusatz oder die Imprägnierung zusätzlich zum Antibiotikum oder alternativ eine entzündungshemmende Substanz umfassen, beispielsweise Hydrocortison.
Gemäß einer besonders bevorzugten Ausführungsform umfasst die antimikrobiell wirksame Substanz Silber oder eine Silber-Verbindung.
Die Wundauflage, also der erste und/oder der zweite Schaumstoff, kann Silber in Form von Silberionen oder in Form von atomarem Silber enthalten. Bevorzugt ist Silber in Form einer Silberbeschichtung auf dem ersten und/oder der zweiten Schaumstoff der Wundauflage aufgebracht. Alternativ kann das Silber innerhalb der Wundauflage, also innerhalb des ersten und/oder des zweiten Schaumstoffs verteilt sein. Beispielsweise kann bei offenzelligen Schaumstoffen Silber bereits in die härtbare Zusammensetzung mit eingebracht werden. Bevorzugt enthält der erste und/oder der zweite Schaumstoff 0,000001 bis 0,1 Gew.-%, mehr bevorzugt 0,0001 bis 0,01 Gew.-% Silber, bezogen auf das Gesamtgewicht des jeweiligen Schaumstoffs. Besonders bevorzugt enthält sowohl der erste als auch der zweite Schaumstoff jeweils 0,0001 bis 0,01 Gew.-% Silber, bezogen auf das Gesamtgewicht des jeweiligen Schaumstoffs.
Des weiteren kann der erste und/oder der zweite Schaumstoff einen Partikel-förmigen Zusatz umfassen, wobei es sich bei dem Partikel-förmigen Zusatz insbesondere um ein quellfähiges Polymer handelt. Gemäß einer bevorzugten Ausführungsform umfasst der erste und/oder der zweite Schaumstoff Partikel aus einem superabsorbierenden Polymer (SAP).

Die erfindungsgemäße Wundauflage ist Bestandteil einer Vorrichtung zur Unterdrucktherapie von Wunden. Die Vorrichtung zur Unterdrucktherapie umfasst weiterhin eine Unterdruckquelle und ein Abdeckmaterial zum luftdichten Verschließen eines Wundraums. Vorzugsweise ist die erfindungsgemäße Wundauflage Bestandteil eines Verband-Sets, bei dem die darin enthaltenen Komponenten herstellerseitig steril bereitgestellt werden. Die einzelnen Komponenten sollten hierbei jeweils separat steril abgepackt vorliegen, wobei die Gesamtheit der Komponenten wiederum in einer einzigen Umverpackung untergebracht sein könnte. Idealerweise erfolgt die Sterilisation nach dem Verpacken der Komponenten in der Umverpackung, beispielsweise unter Verwendung von Ethylenoxid.
Die Vorrichtung zur Unterdrucktherapie kann gleichfalls weitere Verbandkomponenten umfassen, beispielsweise zusätzliche Verbandlagen und/oder Druckverteilungschichten. Die zusätzlichen Verbandlagen können beispielsweise ein oder mehrlagig ausgebildet sein. Insbesondere kann es vorteilhaft sein, wenn die Vorrichtung eine Wundkontaktschicht umfasst, welche zum Einbringen zwischen Wundgrund und der erfindungsgemäßen Wundauflage geeignet ist, beispielsweise eine atraumatisch ausgestaltete, gelochte Folienlage oder eine Wundgaze. Gegenstand der Erfindung ist gleichzeitig ein Verfahren nach Anspruch 15. Das Verfahren umfasst die Schritte
i) Bereitstellen einer ersten Lage aus einem ersten porösen Schaumstoff, beispielsweise eines Schaumstoffs aus Polyester-Polyurethan, Polyether-Polyurethan, Polyorganosiloxan oder Polyvinyl-Alkohol,
ii) Bereitstellen einer zweiten Lage aus einem zweiten porösen Schaumstoff, beispielsweise eines Schaumstoffs aus Polyester-Polyurethan, Polyether-Polyurethan, Polyorganosiloxan oder Polyvinyl-Alkohol,
iii) Herstellen einer unlösbaren Verbindung zwischen erster Lage und zweiter Lage, beispielsweise durch Verkleben, Laminieren oder mechanisches Verbinden,
iv) Einbringen von durchgehenden Öffnungen, welche beide Lagen kanalartig und vollständig durchdringen, beispielsweise durch Bohren, Stanzen oder mittels Hitzeeinwirkung.
Wesentlich bei dem Verfahren ist, dass sich der in Schritt i) bereitgestellte erste Schaumstoff von dem in Schritt ii) bereitgestellten zweiten Schaumstoff hinsichtlich einer der Eigenschaft des Schaumstoffs umfassend die Zellzahl, gemessen in Zellen pro Inch, der Rohdichte, gemessen gemäß DIN EN ISO 845, der Stauchhärte, gemessen nach ISO 3386-1 oder in der Bruchdehnung, gemessen nach DIN 53571, unterscheidet.

Ein weiterer bevorzugter Gegenstand der Erfindung umfasst eine erste und eine zweite Wundauflage, jeweils der erfindungsgemäßen Form, wie vorstehend beschrieben, zur Anwendung in der Unterdrucktherapie von Wunden am menschlichen oder tierischen Körper, dadurch gekennzeichnet,
dass die erste Wundauflage in einer ersten Behandlungsphase zur Reinigung der Wunde angewandt wird, wobei die erste Wundauflage mit ihrer ersten Lage zum Wundgrund hin zeigend eingebracht wird. Der Gegenstand ist weiter dadurch gekennzeichnet, dass die zweite Wundauflage in einer zweiten Behandlungsphase zur Förderung der Granulation mit ihrer zweiten Lage zum Wundgrund hin zeigend eingebracht wird.
Der in diesem Zusammenhang beschriebene Gegenstand kann mindestens eine weitere Wundauflage, jeweils der erfindungsgemäßen Form, wie vorstehend beschrieben, umfassen, zur Anwendung in der Unterdrucktherapie von Wunden am menschlichen oder tierischen Körper, dadurch gekennzeichnet, dass die weitere Wundauflage in einer dritten Behandlungsphase zur Stimulation der Differenzierung- oder Umbauphase angewandt wird, wobei die weitere Wundauflage mit ihrer zweiten Lage zum Wundgrund hin zeigend in die Wunde eingebracht wird.

In jedem der drei Behandlungsphasen (Reinigungsphase, Granulationsphase und Differenzierung- bzw. Umbauphase) können gegebenenfalls ein oder mehrere Verbandwechsel erforderlich sein. Entsprechend der jeweiligen Behandlungsphase wird die erfindungsgemäße Wundauflage entweder mit ihrer ersten Lage zum Wundgrund hin zeigend (in der Reinigungsphase) oder mit ihrer zweiten Lage zum Wundgrund hin zeigend (in der Granulationsphase und Differenzierung- bzw. Umbauphase) eingebracht.
Gegebenenfalls kann sich, wie beispielsweise in der WO2014/048514 beschrieben, an die Unterdruckbehandlung ein weiterer Behandlungsabschnitt anschließen, in welchem die therapeutische Behandlung unter Einsatz einer Wundauflage ohne Erzeugung eines Unterdrucks durchgeführt wird, wobei die Wundauflage einen Saugkörper umfasst, welcher ein quellfähiges Polymer, insbesondere ein superabsorbierendes Polymer, umfasst.

### Ausführungsbeispiel 1

### Erster Schaumstoff (erste Lage):

Hydrophober, retikulierter Polyester-Polyurethan-Schaumstoff. Rohdichte gemäß ISO 845: 26,0 kg/m³, Stauchhärte gemäß DIN EN ISO 3386-1: 3,4 kPa, Bruchdehnung gemäß DIN 53571: 290 %, Zellzahl (mikroskopisch bestimmt an einer an der Oberfläche des Schaumes angelegten geraden Linie): 26 pro Inch.

Die Dicke der ersten Schaumstofflage beträgt 25 mm.

### Zweiter Schaumstoff (zweite Lage):

Hydrophober, retikulierter Polyester-Polyurethan-Schaumstoff. Rohdichte gemäß ISO 845: 28,0 kg/m³, Stauchhärte gemäß DIN EN ISO 3386-1: 3,6 kPa, Bruchdehnung gemäß DIN 53571: 335 %, Zellzahl (mikroskopisch bestimmt an einer an der Oberfläche des Schaumes angelegten geraden Linie): 38 pro Inch.

Die Dicke der zweiten Schaumstofflage beträgt 15 mm.

### Ausführungsbeispiel 2

### Erster Schaumstoff (erste Lage):

Hydrophober Polyester-Polyurethan-Schaumstoff. Rohdichte gemäß ISO 845: 26,0 kg/m³, Stauchhärte gemäß DIN EN ISO 3386-1: 3,4 kPa, Bruchdehnung gemäß DIN 53571: 290 %, Zellzahl (mikroskopisch bestimmt an einer an der Oberfläche des Schaumes angelegten geraden Linie): 26 pro Inch.

Die Dicke der ersten Schaumstofflage beträgt 20 mm.

### Zweiter Schaumstoff (zweite Lage):

Hydrophober Polyether-Polyurethan-Schaumstoff. Rohdichte gemäß ISO 845: 23,0 kg/m³, Stauchhärte gemäß DIN EN ISO 3386-1: 3,8 kPa, Bruchdehnung gemäß DIN 53571: 310 %, Zellzahl (mikroskopisch bestimmt an einer an der Oberfläche des Schaumes angelegten geraden Linie): 31 pro Inch.

Die Dicke der zweiten Schaumstofflage beträgt 20 mm.

### Anwendungsbeispiel I

Dekubitalwunde mit einer großflächigen (Durchmesser ca. 16 cm) und nach außen hin offenen Wundhöhle. Nekrotisches Gewebe, insbesondere im zentralen Bereich. Die Unterdrucktherapie erfolgt unter Verwendung der in Ausführungsbeispiel 1 beschriebenen Wundauflage, wobei die Wundauflage in der ersten Behandlungsphase mit ihrer ersten Lage zum Wundgrund hin zeigend in die Wunde eingebracht wird. Der erste Verbandwechsel erfolgt nach 5 Tagen. Beim erneuten Anlegen des Unterdruck-Verbandes wird eine frische Wundauflage gemäß der in Ausführungsbeispiel 1 beschriebenen Ausführung erneut mit ihrer ersten Lage zum Wundgrund hin zeigend in die Wunde eingebracht. Sobald die Wunde nach Einschätzung des Behandlers in die Granulationsphase eintritt, wird bei allen nachfolgend erforderlichen weiteren Verbandwechseln dann jeweils eine frische Wundauflage gemäß der in Ausführungsbeispiel 1 beschriebene Art mit ihrer zweiten Lage zum Wundgrund hin zeigend in die Wunde eingebracht.

### Anwendungsbeispiel II

### Wunde infolge traumatischer Einwirkung (Durchmesser ca. 9 cm).

Die Unterdrucktherapie erfolgt unter Verwendung der in Ausführungsbeispiel 2 beschriebenen Wundauflage, wobei die Wundauflage in der ersten Behandlungsphase mit ihrer ersten Lage zum Wundgrund hin zeigend in die Wunde eingebracht wird. Der erste Verbandwechsel erfolgt nach 5 Tagen. Beim erneuten Anlegen des Unterdruck-Verbandes wird nun eine frische Wundauflage gemäß der in Ausführungsbeispiel 2 beschriebenen Ausführung mit ihrer zweiten Lage zum Wundgrund hin zeigend in die Wunde eingebracht. Bei den nachfolgend erforderlichen weiteren Verbandwechseln wird gleichfalls jeweils eine frische Wundauflage gemäß der in Ausführungsbeispiel 2 beschriebene Art mit ihrer zweiten Lage zum Wundgrund hin zeigend in die Wunde eingebracht.

### Figurenbeschreibung

- 1: Erste Lage der Wundauflage
- 2: Zweite Lage der Wundauflage
- 3: Durchgehende Öffnunge, welche die erste Lage 1 und die zweite Lage 2 vollständig durchdringt
- 4: Luftundurchlässige Abdeckfolie
- 5: Unterdruck-Anschlussstück (Port)
- 6: Unterdruck-Leitung
- 7: Wundgrund
- 8: Wundumgebung
- 10: Wundauflage
- 20: Unterdruckverband

- Figur 1: Ausführungsform der erfindungsgemäßen Wundauflage in perspektivischer Darstellung
- Figur 2: An eine Wunde angelegten Unterdruck-Verband
- Figur 3 a/b: Ausführungsformen der erfindungsgemäßen Wundauflage in der Aufsicht

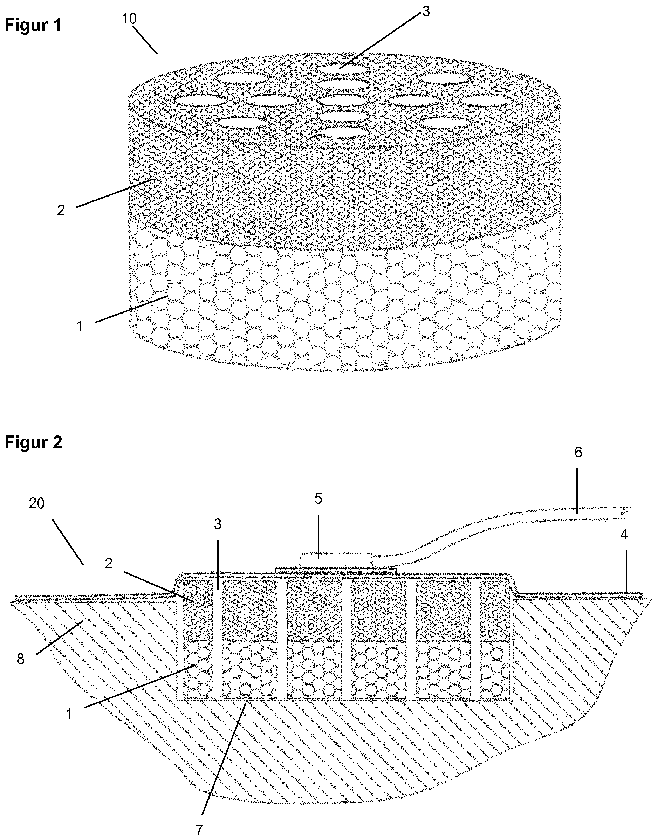

Figur 1 stellt in stark schematisierter Darstellung beispielhaft eine Ausführungsform der erfindungsgemäßen Wundauflage 10 dar. Gezeigt ist eine perspektivische Ansicht, wobei die erste Lage 1 unten und die zweite Lage 2 oben angeordnet ist. Auf der Oberseite der zweiten Lage 2 ist eine Vielzahl von Öffnungen 3 erkennbar, welche beide Lagen vollständig und kanalartig durchdringen (in Figur 1 nicht erkennbar). In dem in Figur 1 dargestellten Beispiel umfasst die Wundauflage 10 eine erste Lage 1 aus einem Schaumstoff, welcher im Vergleich zur zweiten Lage 2 wesentlich größere Poren aufweist. Weiterhin umfasst die Wundauflage eine zweite Lage 2 aus einem zweiten Schaumstoff mit im Vergleich zum ersten Schaumstoff kleineren Poren. Bei dem Schaumstoff, welcher die erste Lage bildet, könnte es sich beispielsweise um den in Ausführungsbeispiel 1 näher beschriebenen ersten hydrophoben Polyester-Polyurethan-Schaumstoff handeln (Zellzahl 26 pro Inch). Bei dem die zweite Lage bildenden Schaumstoff könnte es sich beispielsweise um den in Ausführungsbeispiel 1 näher beschriebenen zweiten hydrophoben Polyester-Polyurethan-Schaumstoff handeln (Zellzahl 38 pro Inch). Erste Lage 1 und zweite Lage 2 sind herstellerseitig unlösbar und dauerhaft miteinander verbunden, beispielsweise durch übliche Laminierungsverfahren. Die in Figur 1 schematisch gezeichnete Wundauflage 10 weist eine runde Form auf. Die Wundauflage 10 kann durch den Anwender vor dem Anlegen des Unterdruckverbandes durch Zuschneiden an Größe und Umriss der zu behandelnden Wunde angepasst werden.

Figur 2 zeigt in stark schematisierter Darstellung beispielhaft einen an eine Wunde angelegten Unterdruck-Verband 20. Die Wunde befindet sich in der Reinigungsphase. Der Unterdruckverband 20 umfasst eine zweilagige Wundauflage, eine luftdichte Abdeckung 4 und ein Unterdruckanschlussstück 5, welches mit einer Unterdruckleitung 6 verbunden ist. Die zweilagige Wundauflage 10 umfasst eine erste Lage 1 und eine zweite Lage 2, wie in Figur 1 dargestellt. Bei dem in Figur 2 gezeigten Beispiel weist der die erste Lage 1 bildende erste Schaumstoff eine im Vergleich zu dem die zweite Lage 2 bildenden zweiten Schaumstoff größere Poren auf. In Figur 2 sichtbar sind die Öffnungen 3, welche die erste Lage 1 und die zweite Lage 2 kanalartig und vollständig durchdringen. Die Öffnungen 3 sind, wie in Figur 2 erkennbar, vorzugsweise vertikal gegenüber der Planebene der Wundauflage angeordnet. Die Wundauflage wurde mit ihrer ersten Lage 1 zum Wundgrund 7 hin zeigend in die Wunde eingebracht. Entsprechend soll die in Figur 1 dargestellte Situation die Unterdruckbehandlung einer Wunde, welche sich in der ersten Heilungsphase (Reinigungsphase) befindet, darstellen. Nach einem oder mehreren Verbandwechseln kann die Wunde in die nachfolgende Granulationsphase übergehen. Die erfindungsgemäße Wundauflage wird dann - anders als in Figur 2 gezeigt - mit ihrer zweiten Lage 2 zum Wundgrund 7 hin zeigend in die Wunde eingebracht. In dem in Figur 2 gezeigten Beispiel befindet sich die Wundauflage in direktem Kontakt mit dem Wundgrund 7. Es ist daher erforderlich, dass es sich sowohl bei dem ersten porösen Schaumstoff als auch bei dem zweiten porösen Schaumstoff um einen zum Kontakt mit dem Wundgrund geeigneten Schaumstoff handelt. Der Schaumstoff muss daher aus einem für medizinische Anwendungen geeigneten Material bestehen. Weiterhin muss der Schaumstoff herstellerseitig steril abgepackt bereitgestellt werden. Grundsätzlich ist es auch möglich, zwischen die erfindungsgemäße Wundauflage und dem Wundgrund 7 eine oder mehrere weitere Verbandlagen einzubringen, beispielsweise eine atraumatische Folie als Wundkontaktschicht (in Figur 2 nicht gezeigt). Gleichfalls ist es möglich, weitere Verbandlagen zwischen die erfindungsgemäße Wundauflage und die luftundurchlässige Abdeckfolie 4 einzubringen, beispielsweise eine oder mehrere Druckverteilungslagen. Die luftundurchlässige Abdeckfolie 4 wird üblicherweise an der die Wunde umgebenden intakten Haut 8 fixiert. Normalerweise ist die Abdeckfolie 4 hierzu mit einer selbstklebenden Beschichtung versehen. Das Unterdruckanschlussmittel 5 kommuniziert über in die Abdeckfolie 4 eingebrachte Öffnungen (in Figur 2 nicht näher dargestellt) mit dem Wundraum. Gleichzeitig ist das Unterdruckanschlussmittel 5 mit einer Unterdruckleitung 6 verbunden, welche ihrerseits an eine Unterdruckquelle (in Figur zwei nicht gezeigt) angeschlossen ist.

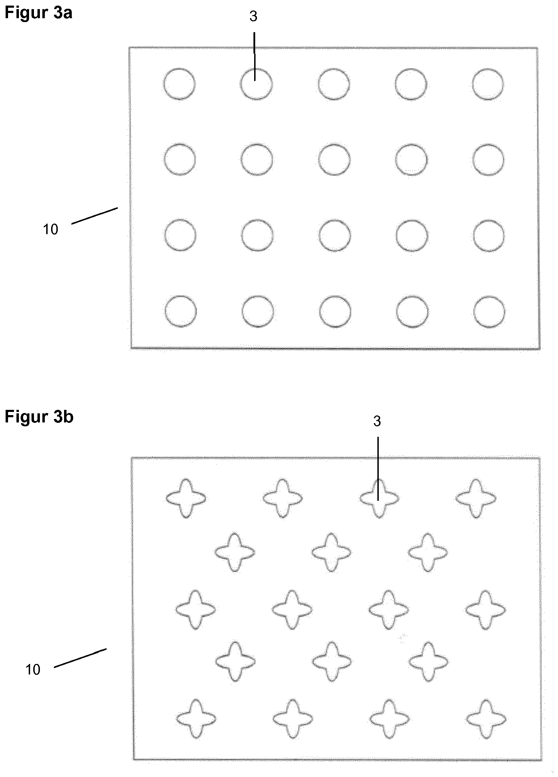

Figur 3 zeigt Beispiele der erfindungsgemäßen Wundauflage 10 in der Aufsicht. Erkennbar sind die die beiden Lagen durchdringenden Öffnungen 3, welche einen hinsichtlich ihrer Form beliebigen äußeren Umriss aufweisen können. Beispielhaft gezeigt sind Öffnungen 3 mit kreisförmigen Umriss (Figur 3a) sowie Öffnungen 3 mit sternförmigem Umriss (Figur 3b). Die Öffnungen sollen jedoch einen Durchmesser von mindestens 1 mm, vorzugsweise mindestens 2 mm aufweisen, um eine ausreichende Durchleitung von Wundexsudat zu gewährleisten. Eine ausreichende Durchleitung von Wundexsudat muss nämlich auch dann sichergestellt werden, wenn das Wundexsudat partikelförmige Bestandteile, wie beispielsweise Verklumpungen, enthält. Partikelförmige Bestandteile im Wundexsudat können insbesondere in der anfänglichen Reinigungsphase vorhanden sein. Gleichzeitig sollen die Öffnungen einen Durchmesser von höchstens 15 mm, vorzugsweise höchstens 10 mm nicht überschreiten, da ansonsten ein gleichmäßiger Kontakt des Schaumstoffs mit dem Wundgrund nicht mehr gewährleistet ist.

## Patentansprüche

1. Vorrichtung zur Unterdrucktherapie von Wunden, umfassend
- eine Unterdruckquelle,
- eine Wundauflage zur Verwendung bei der Unterdrucktherapie von Wunden, umfassend
- eine erste Lage aus einem ersten porösen Schaumstoff,
- eine zweite Lage aus einem zweiten porösen Schaumstoff, wobei die zweite Lage mit der ersten Lage unlösbar verbunden ist und wobei es sich bei dem ersten und dem zweiten Schaumstoff um einen offenzelligen Schaumstoff handelt,
- eine Vielzahl von durchgehenden Öffnungen, welche beide Lagen kanalartig und vollständig durchdringen,
- und ein Abdeckmaterial zum luftdichten Verschließen eines Wundraums, **dadurch gekennzeichnet, dass** sich der erste Schaumstoff hinsichtlich einer der Eigenschaft des Schaumstoffs umfassend
- die Zellzahl, gemessen in Zellen pro Inch
- die Rohdichte, gemessen gemäß DIN EN ISO 845
- die Stauchhärte, gemessen nach ISO 3386-1
- die Bruchdehnung, gemessen nach DIN 53571
von dem zweiten Schaumstoff unterscheidet,
wobei die Wundauflage bei der Anwendung in der Unterdrucktherapie nach Wahl des Anwenders sowohl mit ihrer ersten Lage zum Wundgrund hin zeigend als auch mit ihrer zweiten Lage zum Wundgrund hin zeigend in die Wunde eingebracht werden kann.

2. Vorrichtung zur Unterdrucktherapie von Wunden nach Anspruch 1, wobei sowohl die erste Lage als auch die zweite Lage eine Dicke, gemessen gemäß ISO 1923, von mindestens 5,0 mm und höchstens 30,0 mm aufweist.

3. Vorrichtung zur Unterdrucktherapie von Wunden nach Anspruch 1 oder 2, wobei die Wundauflage aus der ersten Lage und der zweiten Lage besteht.

4. Vorrichtung zur Unterdrucktherapie von Wunden nach einem der vorangehenden Ansprüche, wobei die Öffnungen die beiden Lagen im Wesentlichen vertikal durchdringen.

5. Vorrichtung zur Unterdrucktherapie von Wunden nach einem der vorangehenden Ansprüche, wobei die Öffnungen einen Durchmesser von mindestens 1 mm und höchstens 15 mm aufweisen.

6. Vorrichtung zur Unterdrucktherapie von Wunden nach einem der vorangehenden Ansprüche, wobei die Öffnungen einen kreisrunden, einen ovalen, einen rechteckigen, einen quadratischen oder einen sternförmigen Querschnitt aufweisen.

7. Vorrichtung zur Unterdrucktherapie von Wunden nach einem der vorangehenden Ansprüche, wobei der erste Schaumstoff und/oder der zweite Schaumstoff ein Polymer oder eine Polymermischung ausgewählt aus Polyurethan-Ester, Polyurethan-Ether, Polyvinylalkohol, Polyurethan-Polyharnstoff-Copolymer, Polyorganosiloxan, Polyurethan-Silicon-Copolymer, Polylactide, Chitosan oder Kollagen umfasst.

8. Vorrichtung zur Unterdrucktherapie von Wunden nach einem der vorangehenden Ansprüche 1 bis 6, wobei der erste Schaumstoff ein erstes Polymer oder eine erste Polymermischung ausgewählt aus aus Polyurethan-Ester, Polyurethan-Ether, Polyvinylalkohol, Polyurethan-Polyharnstoff-Copolymer, Polyorganosiloxan, Polyurethan-Silicon-Copolymer, Polylactide, Chitosan oder Kollagen umfasst, und wobei der zweite Schaumstoff ein zweites Polymer oder eine zweite Polymermischung ausgewählt aus Polyurethan-Ester, Polyurethan-Ether, Polyvinylalkohol, Polyurethan-Polyharnstoff-Copolymer, Polyorganosiloxan, Polyurethan-Silicon-Copolymer, Polylactide, Chitosan oder Kollagen umfasst, mit der Maßgabe, dass das erste Polymer oder - falls zutreffend - die erste Polymermischung sich von dem zweiten Polymer oder - falls zutreffend - von der zweiten Polymermischung unterscheidet.

9. Vorrichtung zur Unterdrucktherapie von Wunden nach einem der vorangehenden Ansprüche, wobei der erste und/oder der zweite Schaumstoff eine Zellzahl von mindestens 5 und höchstens 200 Zellen pro Inch aufweist.

10. Vorrichtung zur Unterdrucktherapie von Wunden nach einem der vorangehenden Ansprüche, wobei der erste und/oder der zweite Schaumstoff eine Rohdichte zwischen 10 und 350 kg/m³, gemessen gemäß DIN EN ISO 845, aufweist.

11. Vorrichtung zur Unterdrucktherapie von Wunden nach einem der vorangehenden Ansprüche, wobei der erste und/oder der zweite Schaumstoff eine Stauchhärte von mindestens 1,5 kPa und höchstens 22,5 kPa, gemessen nach ISO 3386-1, aufweist.

12. Vorrichtung zur Unterdrucktherapie von Wunden nach einem der vorangehenden Ansprüche, wobei der erste und/oder der zweite Schaumstoff eine Bruchdehnung von mindestens 100 % und höchstens 350 %, gemessen nach DIN 53571, aufweist.

13. Vorrichtung zur Unterdrucktherapie von Wunden nach einem der vorangehenden Ansprüche, wobei der erste Schaumstoff sich hinsichtlich
- seiner Zellzahl, gemessen in Zellen pro Inch
- und/oder seiner Rohdichte, gemessen gemäß DIN EN ISO 845
- und/oder seiner Stauchhärte, gemessen nach ISO 3386-1
- und/ oder seiner Bruchdehnung, gemessen nach DIN 53571 um mindestens den Faktor 1,1 und um höchstens den Faktor 20,0 von dem zweiten Schaumstoff unterscheidet.

14. Vorrichtung zur Unterdrucktherapie von Wunden nach einem der vorangehenden Ansprüche, wobei der erste und/oder der zweite Schaumstoff mit einem Zusatz und/oder Hilfsstoff beschichtet oder imprägniert ist.

15. Verfahren zum Herstellen einer Vorrichtung zur Unterdrucktherapie von Wunden nach einem der vorangehenden Ansprüche 1 bis 14, umfassend
- Bereitstellen einer ersten Lage aus einem ersten, porösen Schaumstoff,
- Bereitstellen einer zweiten Lage aus einem zweiten porösen Schaumstoff,
- Herstellen einer unlösbaren Verbindung zwischen erster Lage und zweiter Lage
- Einbringen von durchgehenden Öffnungen, welche beide Lagen kanalartig und vollständig durchdringen.

## Claims

1. Device for the vacuum therapy of wounds, comprising
- a vacuum source
- a wound dressing for use in vacuum therapy of wounds, comprising
- a first layer of a first porous foam material,
- a second layer of a second porous foam material, wherein the second layer is connected non-detachably to the first layer, and wherein the first and the second foam material is an open-cell foam material
- a plurality of continuous openings, which penetrate both layers in a channel-like manner and completely,
- and a covering material for airtight closing of a wound cavity,
**characterized in that** the first foam material differs from the second foam material with regard to a characteristic of the foam material comprising
- the cell count, measured in cells per inch
- the bulk density, measured according to DIN EN ISO 845
- the compression hardness, measured according to ISO 3386-1
- the elongation at break, measured according to DIN 53571,
wherein the wound dressing, with use in vacuum therapy, can be introduced into the wound both with its first layer pointing towards the wound base and also with its second layer pointing towards the wound base, according to the choice of the user.

2. Device for the vacuum therapy of wounds according to Claim 1, wherein both the first layer and also the second layer has a thickness, measured according to ISO 1923, of at least 5.0 mm and at most of 30.0 mm.

3. Device for the vacuum therapy of wounds according to Claim 1 or 2, wherein the wound dressing consists of the first layer and of the second layer.

4. Device for the vacuum therapy of wounds according to any of the preceding claims, wherein the openings penetrate the two layers substantially vertically.

5. Device for the vacuum therapy of wounds according to any of the preceding claims, wherein the openings have a diameter of at least 1 mm and at most 15 mm.

6. Device for the vacuum therapy of wounds according to any of the preceding claims, wherein the openings have a circular, an oval, a rectangular, a square or a star-shaped cross section.

7. Device for the vacuum therapy of wounds according to any of the preceding claims, wherein the first foam material and/or the second foam material comprises a polymer or a polymer mixture selected from polyurethane ester, polyurethane ether, polyvinyl alcohol, polyurethane-polyurea copolymer, polyorganosiloxane, polyurethane-silicone copolymer, polylactides, chitosan or collagen.

8. Device for the vacuum therapy of wounds according to any of the preceding Claims 1 to 6, wherein the first foam material comprises a first polymer or a first polymer mixture selected from polyurethane ester, polyurethane ether, polyvinyl alcohol, polyurethane-polyurea copolymer, polyorganosiloxane, polyurethane-silicone copolymer, polylactides, chitosan or collagen, and wherein the second foam material comprises a second polymer or a second polymer mixture selected from polyurethane ester, polyurethane ether, polyvinyl alcohol, polyurethane-polyurea copolymer, polyorganosiloxane, polyurethane-silicone copolymer, polylactides, chitosan or collagen, with the proviso that the first polymer or - if applicable - the first polymer mixture differs from the second polymer or - if applicable - from the second polymer mixture.

9. Device for the vacuum therapy of wounds according to any of the preceding claims, wherein the first and/or the second foam material has a cell count of at least 5 and at most 200 cells per inch.

10. Device for the vacuum therapy of wounds according to any of the preceding claims, wherein the first and/or the second foam material has a bulk density between 10 and 350 kg/m³, measured according to DIN EN ISO 845.

11. Device for the vacuum therapy of wounds according to any of the preceding claims, wherein the first and/or the second foam material has a compression hardness of at least 1.5 kPa and at most 22.5 kPa, measured according to ISO 3386-1.

12. Device for the vacuum therapy of wounds according to any of the preceding claims, wherein the first and/or the second foam material has an elongation at break of at least 100 % and at most of 350 %, measured according to DIN 53571.

13. Device for the vacuum therapy of wounds according to any of the preceding claims, wherein the first foam material differs from the second foam material with regard to
- its cell number, measured in cells per inch
- and/or its bulk density, measured according to DIN EN ISO 845
- and/or its compression hardness, measured according to ISO 3386-1
- and/or its elongation at break, measured according to DIN 53571
by at least the factor 1.1 and by at most the factor 20.0.

14. Device for the vacuum therapy of wounds according to any of the preceding claims, wherein the first and/or the second foam material is coated or impregnated with an addition and/or adjuvant.

15. Method for producing a device for the vacuum therapy of wounds according to any of preceding Claims 1 to 14, comprising
- providing a first layer of a first, porous foam material,
- providing a second layer of a second porous foam material,
- producing a non-detachable connection between first layer and second layer
- introducing continuous openings which penetrate both layers in a channel-like manner and completely.

## Revendications

1. Dispositif pour un traitement par pression négative de plaies, comprenant :
- une source de pression négative,
- un pansement pour une utilisation lors de la thérapie par pression négative de plaies, comprenant
- une première couche d'une première mousse poreuse,
- une deuxième couche d'une deuxième mousse poreuse, la deuxième couche étant reliée de manière non amovible avec la première couche et dans lequel la première et la deuxième mousse sont une mousse à cellules ouvertes,
- une pluralité d'ouvertures continues qui traversent entièrement les deux couches sous la forme de canaux,
- et un matériau de recouvrement pour la fermeture étanche à l'air d'un espace de plaie,
**caractérisé en ce que** la première mousse diffère de la deuxième mousse au regard d'une propriété de la mousse comprenant
- le nombre de cellules, mesuré en cellules par pouce,
- la densité brute, mesurée selon DIN EN ISO 845,
- la résistance à la compression, mesurée selon ISO 3386-1,
- l'allongement à la rupture, mesuré selon DIN 53571, le pansement pouvant être disposé dans la plaie lors de l'utilisation dans le traitement par pression négative au choix de l'utilisateur aussi bien avec sa première couche orientée vers le lit de la plaie qu'avec sa deuxième couche orientée vers le lit de la plaie.

2. Dispositif pour un traitement par pression négative de plaies selon la revendication 1, dans lequel aussi bien la première couche que la deuxième couche présentent une épaisseur, mesurée selon ISO 1923, d'au moins 5,0 mm et d'au plus 30,0 mm.

3. Dispositif pour un traitement par pression négative de plaies selon la revendication 1 ou 2, dans lequel le pansement est constitué par la première couche et la deuxième couche.

4. Dispositif pour un traitement par pression négative de plaies selon l'une quelconque des revendications précédentes, dans lequel les ouvertures traversent essentiellement verticalement les deux couches.

5. Dispositif pour un traitement par pression négative de plaies selon l'une quelconque des revendications précédentes, dans lequel les ouvertures présentent un diamètre d'au moins 1 mm et d'au plus 15 mm.

6. Dispositif pour un traitement par pression négative de plaies selon l'une quelconque des revendications précédentes, dans lequel les ouvertures présentent une section transversale ronde, ovale, rectangulaire, carrée ou en étoile.

7. Dispositif pour un traitement par pression négative de plaies selon l'une quelconque des revendications précédentes, dans lequel la première mousse et/ou la deuxième mousse comprend un polymère ou un mélange de polymères choisis parmi les esters de polyuréthane, les éthers de polyuréthane, l'alcool polyvinylique, le copolymère de polyuréthane-polyurée, le polyorganosiloxane, le copolymère de polyuréthane-silicone, le polylactide, le chitosane ou le collagène.

8. Dispositif pour un traitement par pression négative de plaies selon l'une quelconque des revendications 1 à 6 précédentes, dans lequel la première mousse comprend un premier polymère ou un premier mélange de polymères choisis parmi les esters de polyuréthane, les éthers de polyuréthane, l'alcool polyvinylique, le copolymère de polyuréthane-polyurée, le polyorganosiloxane, le copolymère de polyuréthane-silicone, le polylactide, le chitosane ou le collagène, et la deuxième mousse comprend un deuxième polymère ou un deuxième mélange de polymères choisis parmi les esters de polyuréthane, les éthers de polyuréthane, l'alcool polyvinylique, le copolymère de polyuréthane-polyurée, le polyorganosiloxane, le copolymère de polyuréthane-silicone, le polylactide, le chitosane ou le collagène, à condition que le premier polymère, ou le cas échéant le premier mélange de polymères, diffère du deuxième polymère, ou le cas échéant du deuxième mélange de polymères.

9. Dispositif pour un traitement par pression négative de plaies selon l'une quelconque des revendications précédentes, dans lequel la première et/ou la deuxième mousse présentent un nombre de cellules d'au moins 5 et d'au plus 200 cellules par pouce.

10. Dispositif pour un traitement par pression négative de plaies selon l'une quelconque des revendications précédentes, dans lequel la première et/ou la deuxième mousse présentent une densité brute comprise entre 10 et 350 kg/m³, mesurée selon DIN EN ISO 845.

11. Dispositif pour un traitement par pression négative de plaies selon l'une quelconque des revendications précédentes, dans lequel la première et/ou la deuxième mousse présentent une résistance à la compression d'au moins 1,5 kPa et d'au plus 22,5 kPa, mesurée selon ISO 3386-1.

12. Dispositif pour un traitement par pression négative de plaies selon l'une quelconque des revendications précédentes, dans lequel la première et/ou la deuxième mousse présentent un allongement à la rupture d'au moins 100 % et d'au plus 350 %, mesuré selon DIN 53571.

13. Dispositif pour un traitement par pression négative de plaies selon l'une quelconque des revendications précédentes, dans lequel la première mousse diffère de la deuxième mousse au regard de
- son nombre de cellules, mesuré en cellules par pouce
- et/ou sa densité brute, mesurée selon DIN EN ISO 845,
- et/ou sa résistance à la compression, mesurée selon ISO 3386-1,
- et/ou son allongement à la rupture, mesuré selon DIN 53571,
d'au moins un facteur de 1,1 et d'au plus un facteur de 20,0.

14. Dispositif pour un traitement par pression négative de plaies selon l'une quelconque des revendications précédentes, dans lequel la première et/ou la deuxième mousse sont revêtues ou imprégnées avec un additif et/ou un adjuvant.

15. Procédé de fabrication d'un dispositif pour un traitement par pression négative de plaies selon l'une quelconque des revendications précédentes 1 à 14, comprenant :
- la préparation d'une première coche d'une première mousse poreuse,
- la préparation d'une deuxième couche d'une deuxième mousse poreuse,
- la création d'une liaison non amovible entre la première couche et la deuxième couche,
- la mise en place d'ouvertures continues qui traversent entièrement les deux couches sous la forme de canaux.
